(19) Europäisches Patentamt

European Patent Office

Office européen des brevets

(11) Veröffentlichungsnummer: **0 288 919 B1**

(12) **EUROPÄISCHE PATENTSCHRIFT**

(45) Veröffentlichungstag der Patentschrift: **04.03.92**

(21) Anmeldenummer: **88106472.9**

(22) Anmeldetag: **22.04.88**

(51) Int. Cl.⁵: **A61K 7/50**

(54) **Gelförmiges Schaumbadkonzentrat.**

(30) Priorität: **30.04.87 DE 3714455**

(43) Veröffentlichungstag der Anmeldung:
**02.11.88 Patentblatt 88/44**

(45) Bekanntmachung des Hinweises auf die
Patenterteilung:
**04.03.92 Patentblatt 92/10**

(84) Benannte Vertragsstaaten:
**AT BE CH DE ES FR GB IT LI NL**

(56) Entgegenhaltungen:
**EP-A- 0 037 009**
**EP-A- 0 167 382**
**EP-A- 0 217 250**
**US-A- 4 426 310**

(73) Patentinhaber: **Henkel Kommanditgesellschaft
auf Aktien
Postfach 1100 Henkelstrasse 67
W-4000 Düsseldorf-Holthausen(DE)**

(72) Erfinder: **Bitter, Ingrid
Friedrich-Ebert-Strasse 201
W-4150 Krefeld(DE)**
Erfinder: **Scholz, Wolfhard
Edmundstrasse 26
W-4150 Krefeld 11(DE)**
Erfinder: **Schneider, Werner, Dr.
Uerdinger Strasse 467
W-4150 Krefeld-Bockum(DE)**

**Beschreibung**

Die Erfindung betrifft ein gelförmiges Schaumbadkonzentrat mit hohem Tensid- und Duftstoffgehalt.

Schaumbademittel werden dem Badewasser zugesetzt und haben die Aufgabe, einen voluminösen, feinblasigen Schaum zu entwickeln, dem Badewasser einen angenehmen Duft zu verleihen und die Reinigung des Körpers im Badewasser zu unterstützen. Dabei soll die Haut nicht durch eine zu starke entfettende Wirkung der Tenside geschädigt werden. Es wurde daher oft vorgeschlagen, durch geeignete Zusätze, z. B. durch kosmetische Ölkomponenten, rückfettende oder hautfeuchthaltende Wirkstoffe, wasserlösliche Proteine oder Proteinabbauprodukte nicht nur der entfettenden Wirkung der Tenside entgegenzuwirken, sondern zusätzlich eine kosmetische Verbesserung des Hautzustandes zu erreichen.

Handelsübliche Produkte enthalten 10 bis 30 Gew.-% Waschaktivsubstanz und werden mit 20 bis 40 g pro Vollbad (von ca. 200 l) dosiert. Produkte mit einer höheren Waschaktivsubstanzkonzentration weisen den Vorteil geringerer Verpackungskosten, geringeren Konservierungsmittelbedarfs und kostengünstigerer Lagerhaltung auf. Es sind auch bereits hochkonzentrierte und sogar wasserfreie Produkte bekannt, die aber noch zahlreiche Nachteile aufweisen:

DE-OS 29 43 202 beschreibt Pflegeschaumbademittel mit 20 bis 80 Gew.-% oberflächenaktiver Substanzen und 20 bis 80 Gew.-% kosmetischen Ölen. Auch DE-OS 27 00 891 beschreibt Badezusätze, die 40 bis 75 Gew.-% oberflächenaktiver Substanzen und 15 bis 50 Gew.-% kosmetischer Öle enthalten. Der hohe Tensidgehalt dieser Produkte dient dazu, die kosmetischen Öle weitgehend zu solubilisieren bzw. in Wasser dispergierbar zu machen. Die Produkte befriedigen aber nicht im Hinblick auf das Schäumvermögen, und sie hinterlassen auf der Haut merklich ölige Rückstände. Andere hochkonzentrierte Tensidzusammensetzungen haben den Nachteil, daß sie steife Pasten darstellen oder daß sich beim Versuch, die Produkte in Wasser aufzulösen, schwer auflösbare, feste Gelpartikei bilden, was man durch Zusatz von Lösungsmitteln oder Lösungsvermittlern zu beheben sucht.

EP-A-0 217 250 beschreibt Badezusätze mit einem Tensid gehalt bis zu 60 % sowie einem Anteil an klar solubilisiertem Öl zur Fettung und Pflege der Haut.

Es bestand daher die Aufgabe, konzentrierte Schaumbadezusätze zu finden, die einerseits einen reichhaltigen Schaum entwickeln, die hohe Parfümölmengen solubilisieren und die weder eine schädigende Wirkung auf die Haut ausüben noch einen unangenehmen öligen Film auf der Haut hinterlassen und die sich im Badewasser leicht und rasch auflösen. Dieses Ziel wurde in hohem Maße erreicht durch das erfindungsgemäße Schaumbadkonzentrat, welches gekennzeichnet ist durch einen Gehalt von

(A) 35 bis 45 Gew.-% Alkylethersulfat-Tenside der Formel $R^1$-O$(C_2H_4O)_n$-SO$_3$Na, in der $R^1$ eine Alkylgruppe mit 12 bis 16 C-Atomen und n = 1 bis 6 ist,

(B) 5 bis 15 Gew.-% eines oder mehrerer nichtionogener Polyglykolethertenside der Formel $R^2$-A-$(C_2H_4O)_x$-H, in der $R^2$ eine Alkyl-, Alkenyl- oder Acylgruppe mit 12 bis 18 C-Atomen, A = Sauerstoff oder eine -NH-Gruppe und x = 3 bis 9 ist,

(C) 1 bis 5 Gew.-% eines oder mehrerer amphoterer oder zwitterionischer Tenside,

(D) 1 bis 5 Gew.-% eines Glycerinpolyglykolether-Monofettsäureesters oder eines Fettsäuremono- oder diglycerid-polyglykolethers auf Basis einer Fettsäure mit 12 bis 18 C-Atomen und mit jeweils 5 bis 20 Glykolethergruppen,

(E) 2 bis 10 Gew.-% eines wasserunlöslichen Duftstofföls und

30 bis 50 Gew.-% Wasser.

Die ausgewogene Komposition von hochkonzentriertem Alkylethersulfat, nichtionischem Tensid, amphoterem bzw. zwitterionischem Tensid und hydrophilem Öl führt zu flüssigen Badezusätzen, die aufgrund starker Strukturviskosität einen gelförmigen Eindruck machen. Dies bedeutet, daß die erfindungsgemäßen Schaumbadkonzentrate sehr hochviskos und nicht frei fließbar erscheinen, daß sie aber unter dem Einfluß von Scherkräften sehr gut fließen und sich völlig problemlos aus flexiblen Tuben und flexiblen Flaschen ausdrücken oder aus Kolbendosierspendern sehr gut dosieren lassen. Darüber hinaus lösen sich die erfindungsgemäßen Schaumbadkonzentrate rasch und ohne Gelbildung im Badewasser und bewirken eine stabile, gleichmäßige Verteilung der Duftstoff-Öle und der Farbstoffe. Die Haut wird auf schonende Weise gereinigt, so daß die gegebenenfalls in der Zusammensetzung enthaltenen pflegenden Wirkstoffe voll zu Entfaltung kommen.

Die Alkylethersulfat-Tenside der Formel $R^1$-O$(C_2H_4O)_n$-SO$_3$Na sind bekannte Produkte des Handels. Sie werden hergestellt aus Fettalkoholen mit 12 bis 16 C-Atomen, bevorzugt mit linearer Alkylkette, durch Anlagerung von 1 bis 6 Mol Ethylenoxid und Sulfatierung der dabei erhaltenen Oxethylate mit Schwefeltrioxid oder Chlorsulfonsäure. Die dabei erhaltenen Schwefelsäurehalbester der Fettalkoholoxethylate werden dann mit wäßrigem Natriumhydroxid neutralisiert. Wegen der erforderlichen hohen Konzentration an Alkylethersulfat-Tensid im Produkt können hierfür nicht die handelsüblichen 25- bis 30Gew.-%igen wäßrigen Lösungen sondern die 60- bis 70Gew.-%igen fließfähigen bis gelförmigen Lösungen eingesetzt werden. Solche Produkte sind ebenfalls handelsüblich (z. B. Texapon$^{(R)}$N70, Henkel KGaA).

Die nichtionogenen Polyglykolether-Tenside der Formel $R^2$-A-$(C_2H_4O)_x$-H sind ebenfalls im Handel erhältliche Produkte. Sie werden hergestellt durch Anlagerung von x = 3 bis 9 Mol Ethylenoxid an gesättigte und ungesättigte Fettalkohole, Fettamine, Fettsäuren und/oder Fettsäureamide mit jeweils 12 bis 18 C-Atomen oder durch Anlagerung von 2 bis 8 Mol Ethylenoxid an Fettsäure-$(C_{12}$-$C_{18})$-monoethanolamide.

Als amphotere Tenside werden solche verstanden, die neben einer Alkyl- oder Acylgruppe mit 12 bis 18 C-Atomen eine Aminogruppe und eine Carbonsäure- oder Sulfonsäuregruppe im Molekül tragen. Beispiele für solche amphotere Tenside sind z. B. N-$(C_{12}$-$C_{18}$-alkyl)-$\beta$-aminopropionsäure, N-$(C_8$-$C_{18}$-alkyl)-$\beta$-aminobuttersäure, N-$(C_8$-$C_{18}$-alkyl)-iminodiessigsäure, N-$(C_{12}$-$C_{16}$-alkyl)-N-2-hydroxyethyl-glycin, N-$(C_{12}$-$C_{18}$-alkyl)-taurin, N-$(C_8$-$C_{18}$-alkyl)-benzylamin-p-sulfonsäure, N-$(C_8$-$C_{18}$-alkyl)-1-aminobutan-3-sulfonsäure.

Als zwitterionische Tenside sind solche zu verstehen, die neben einer Alkyl- oder Acylgruppe mit 12 bis 18 C-Atomen eine quartäre Ammoniumgruppe und eine Carboxylat- oder Sulfonatgruppe im Molekül enthalten. Beispiele für solche zwitterionischen Tenside sind z. B. N-$(C_8$-$C_{18}$-alkyl)-N,N-dimethyl-ammonioglycinat, N-Kokosacylamidopropyl-N,N-dimethyl-ammonioglycinat, N-$(C_8$-$C_{18}$alkyl)-N-2-hydroxyethyl-N-methyl-$\beta$-ammoniopropionat, N-$(C_8$-$C_{18}$-alkyl)-N,N-dimethyl-$\beta$-ammoniopropansulfonat.

Glycerinpolyglykolether-Monofettsäureester sind hydrophile Ölkomponenten mit hautrückfettender Wirkung. Produkte dieser Struktur sind literaturbekannt, z. B. aus DE-A 20 24 051 und im Handel, z. B. unter dem Warenzeichen Cetiol$^{(R)}$HE (Henkel KGaA) erhältlich. Fettsäuremono- oder Diglycerid-polyglykolether sind ebenfalls als rückfettende Komponenten literaturbekannt, z. B. aus US 2,617,754 und aus DE-A 14 67 816.

Die Kombination der Komponenten (A), (B), (C) und (D) in den spezifizierten Mengen ist geeignet, 2 bis 10 Gew.-% wasserunlöslicher Duftstofföle (E) klar zu lösen und bei der Auflösung in Wasser homogen im Wasser zu verteilen, ohne daß es zu einer Beeinträchtigung des Schaumes oder des Hautgefühls kommt. Als wasserunlösliche Duftstofföle können als für die Parfümierung von Körperpflegemitteln geeigneten ätherischen Öle und synthetischen Riechstoffe und Mischungen dieser Stoffe verwendet werden.

Neben den genannten Komponenten können die erfindungsgemäßen Schaumbadkonzentrate noch übliche Hilfs- und Zusatzmittel in kleineren Mengen bis zu höchstens 5 Gew.-% der Zusammensetzung enthalten. Solche Hilfs- und Zusatzstoffe sind z. B. Konservierungsstoffe, Farbstoffe, Elektrolyte (z. B. Natriumchlorid, Magnesiumsulfat), Polyethylenglykole, kationische Polymere, Vitamine, Pflanzenextrakte und andere hautkosmetische Wirkstoffe.

Eventuell vorhandene weitere wasserlösliche Komponenten können zusammen mit dem Wasser oder zum Schluß zugesetzt werden. Öllösliche Komponenten sollten zusammen mit dem Parfümöl in dem Gemisch aus Komponente (B) und (D) aufgelöst werden.

Von den vorstehend genannten Komponenten (A), (B), (C) und (D) sind spezielle Produkte bzw. Produktgruppen in besonderer Weise zur Kombination miteinander und Herstellung erfindungsgemäßer Schaumbadkonzentrate geeignet, die in bezug auf ihre anwendungstechnischen Eigenschaften, insbesondere in bezug auf das Schäumvermögen, die Hautverträglichkeit und die Auflösbarkeit in Wasser, optimale Eigenschaften aufweisen. Eine solche, besonders bevorzugte Ausführungsform der Erfindung besteht in einem Schaumbadkonzentrat, welches einen Gehalt von

(A1) 35 bis 40 Gew.-% Alkylethersulfat-Tensid der Formel $R^1$-O-$(C_2H_4O)_n$-$SO_3Na$, in der $R^1$ eine Alkylgruppe mit 12 bis 16 C-Atomen und n = 1 bis 3 ist,

(B1) 2 bis 6 Gew.-% eines nichtionogenen Polyglykolethertensids der Formel $R^2$-O$(C_2H_4O)_x$-H, in der $R^2$ eine Alkyl- oder Alkenylgruppe mit 16 bis 18 C-Atomen und x = 3 bis 6 ist,

(B2) 4 bis 8 Gew.-% eines nichtionogenen Polyglykolethertensids der Formel $R^3$CONH$(C_2H_4O)_x$-Hist, in der $R^3$CO eine Acylgruppe mit 12 bis 18 C-Atomen und x = 3 bis 6 ist,

(C) 2 bis 4 Gew.-% eines zwitterionischen Tensids der Formel $R^4$CONH-$(CH_2)_3$-$\overset{(+)}{N}(CH_3)_2CH_2COO^{(-)}$, in welcher $R^4$CO eine Acylgruppe mit 12 bis 18 C-Atomen ist,

(D) 2 bis 4 Gew.-% eines Fettsäure$(C_{12}$-$C_{18})$-monoesters eines Anlagerungsproduktes von 7 bis 10 Mol Ethylenoxid an Glycerin,

(E) 4 bis 6 Gew.-% eines wasserunlöslichen Duftstofföls und

35 bis 45 Gew.-% Wasser

aufweist.

Die Herstellung der erfindungsgemäßen Schaumbadkonzentrate kann auf einfache Weise durch Vermischen der Komponenten bei Raumtemperatur, das heißt ohne Wärmezufuhr erfolgen. Es hat sich aber als vorteilhaft erwiesen, wenn bei dem Vermischen der Komponenten eine besondere Reihenfolge eingehalten wird. Auf diese Weise wird die Bildung schwer auflösbarer, gelförmiger Partikel vermieden und eine rasche, homogene Vermischung der Komponenten erreicht. Bevorzugt arbeitet man so, daß die Komponenten (B) und (D) vorgelegt, das Parfümöl (E) darin aufgelöst und dann nacheinander die Komponenten (C), Wasser und zuletzt die Komponenten (A) zugegeben und jeweils unter Rühren homogen eingemischt werden.

Die erfindungsgemäßen Schaumbadkonzentrate weisen eine hohe Strukturviskosität auf und erscheinen gelförmig, lassen sich aber unter dem Einfluß von Scherkräften gut rühren, pumpen, abfüllen und aus flexiblen Behältern entleeren. Die Viskosität der Produkte liegt üblicherweise bei 15 bis 40 Pa.s (20 °C) (gemessen mit dem Rotationsviskosimeter Rotovisco[R] (Fa. Haake), Type RV3, Maßsystem MV2 bei 0,4 bis 1 U/min.).

Die folgenden Beispiele sollen den Gegenstand der Erfindung näher erläutern, ohne ihn hierauf zu beschränken.

<u>B e i s p i e l e</u>

Gelförmiges Schaumbadkonzentrat

| Zusammensetzung | 1 | 2 |
|---|---|---|
| Texapon(R)N70 | 52 Gew.-% | 57 Gew.-% |
| Eumulgin(R)O5 | 5 Gew.-% | 3 Gew.-% |
| Eumulgin(R)C4 | 5 Gew.-% | 7 Gew.-% |
| Dehyton(R)K | 2,5 Gew.-% | 3,5 Gew.-% |
| GOF* | 2,5 Gew.-% | 3,5 Gew.-% |
| Parfümöl | 5 Gew.-% | 5 Gew.-% |
| Citronensäure . $H_2O$ | 0,13 Gew.-% | 0,13 Gew.-% |
| Wasser | ad 100 Gew.-% | ad 100 Gew.-% |

| | | |
|---|---|---|
| Aussehen | opakes Gel | opakes Gel |
| Viskosität (20 °C) | 12 [Pa.s] | 24 [Pa.s] |

(Haake Rotovisco RV3, Meßsystem MV2 gerippt, 1 UpM)

*GOF: Glycerinoxethylat-fettsäureester    (Kokosfettsäuremonoester
      eines Anlagerungsproduktes von 7 Mol Ethylenoxid an Gly-
      cerin)

Texapon(R)N70: 70%ige wäßrige Lösung eines Alkylethersulfat-
               Natriumsalzes auf Basis eines Anlagerungsproduk-
               tes von 2 Mol Ethylenoxid an ein lineares $C_{12}$-
               $C_{14}$-Fettalkoholgemisch (70 : 30)

Eumulgin(R)C4: Anlagerungsprodukt von 4 Mol Ethylenoxid an Ko-
               kosfettsäure($C_{12}$-$C_{18}$)-monoethanolamid

Eumulgin(R)O5: Anlagerungsprodukt von 5 Mol Ethylenoxid an ein
               Oleyl-Cetlyalkoholgemisch

Dehyton$^{(R)}$K: 30%ige wäßrige Lösung eines N-Kokosacylaminopro-
pyl-N,N-dimethyl-ammoniumglycinat

**Patentansprüche**

1. Gelförmiges Schaumbadkonzentrat, gekennzeichnet durch einen Gehalt von

(A) 35 bis 45 Gew.-%Alkylethersulfat-Tenside der Formel $R^1$-O($C_2H_4O)_n$-$SO_3$Na, in der $R^1$ eine Alkylgruppe mit 12 bis 16 C-Atomen und n = 1 bis 6 ist,
(B) 5 bis 15 Gew.-%eines oder mehrerer nichtionogener Polyglykolethertenside der Formel $R^2$-A-($C_2H_4O)_x$-H, in der $R^2$ eine Alkyl-, Alkenyl- oder Acylgruppe mit 12 bis 18 C-Atomen, A = Sauerstoff oder eine -NH-Gruppe und x = 3 bis 9 ist,
(C) 1 bis 5 Gew.-%eines oder mehrerer amphoterer oder zwitterionischer Tenside,
(D) 1 bis 5 Gew.-%eines Glycerinpolyglykolether-Monofettsäureesters oder eines Fettsäuremono- oder diglycerid-polyglykolethers auf Basis einer Fettsäure mit 12 bis 18 C-Atomen und mit jeweils 5 bis 20 Glykolethergruppen,
(E) 2 bis 10 Gew.-%eines wasserunlöslichen Duftstofföls und
30 bis 50 Gew.-%Wasser.

2. Gelförmiges Schaumbadkonzentrat nach Anspruch 1, gekennzeichnet durch einen Gehalt von

(A) 35 bis 40 Gew.-%Alkylethersulfat-Tensid der Formel $R^1$-O-($C_2H_4O)_n$-$SO_3$Na, in der $R^1$ eine Alkylgruppe mit 12 bis 16 C-Atomen und n = 1 bis 3 ist,
(B1) 2 bis 6 Gew.-%eines nichtionogenen Polyglykolethertensids der Formel $R^2$-O($C_2H_4O)_x$-H, in der $R^2$ eine Alkyl- oder Alkenylgruppe mit 16 bis 18 C-Atomen und x = 3 bis 6 ist,
(B2) 4 bis 8 Gew.-%eines nichtionogenen Polyglykolethertensids der Formel $R^3CONH(C_2H_4O)_x$-Hist, in der $R^3$CO eine Acylgruppe mit 12 bis 18 C-Atomen und x = 3 bis 6 ist,
(C) 2 bis 4 Gew.-%eines zwitterionischen Tensids der Formel $R^4CONH$-$(CH_2)_3$-$\overset{(+)}{N}(CH_3)_2CH_2COO^{(-)}$, in welcher $R^4$CO eine Acylgruppe mit 12 bis 18 C-Atomen ist,
(D) 2 bis 4 Gew.-%eines Fettsäure($C_{12}$-$C_{18}$)-monoesters eines Anlagerungsproduktes von 7 bis 10 Mol Ethylenoxid an Glycerin,
(E) 4 bis 6 Gew.-%eines wasserunlöslichen Duftstofföls und
35 bis 45 Gew.-%Wasser

**Claims**

1. A gel-form foam bath concentrate, characterized by a content of

(A) 35 to 45% by weight alkyl ether sulfate surfactants corresponding to the formula $R^1$-O($C_2H_4O)_n$-$SO_3$Na in which $R^1$ is a $C_{12-16}$ alkyl group and n = 1 to 6,
(B) 5 to 15% by weight of one or more nonionic polyglycol ether surfactants corresponding to the formula $R^2$-A-($C_2H_4O)_x$-H, in which $R^2$ is a $C_{12-18}$ alkyl, alkenyl or acyl group, A is oxygen or an -NH- group and x = 3 to 9,
(C) 1 to 5% by weight of one or more amphoteric or zwitterionic surfactants,
(D) 1 to 5% by weight of a glycerol polyglycol ether monofatty acid ester or of a fatty acid mono- or diglyceride polyglycol ether based on a $C_{12-18}$ fatty acid containing 5 to 20 glycol ether groups in either case,
(E) 2 to 10% by weight of a water-insoluble perfume oil and
30 to 50% by weight water.

2. A gel-form foam bath concentrate as claimed in claim 1, characterized by a content of

(A) 35 to 40% by weight alkyl ether sulfate surfactant corresponding to the formula $R^1$-O($C_2H_4O)_n$-$SO_3$Na in which $R^1$ is a $C_{12-16}$ alkyl group and n = 1 to 3,

(B1) 2 to 6% by weight of a nonionic polyglycol ether surfactant corresponding to the formula $R^2$-O-$(C_2H_4O)_x$-H, in which $R^2$ is a $C_{16-18}$ alkyl or alkenyl group group and x = 3 to 6,

(B2) 4 to 8% by weight of a nonionic polyglycol ether surfactant corresponding to the formula $R^3CONH(C_2H_4O)_x$-H, in which $R^3CO$ is a $C_{12-18}$ acyl group and x = 3 to 6,

(C) 2 to 4% by weight of a zwitterionic surfactant corresponding to the formula $R^4CONH$-$(CH_2)_3$-$N^{(+)}(CH_3)_2CH_2COO^{(-)}$, in which $R^4CO$ is a $C_{12-18}$ acyl group,

(D) 2 to 4% by weight of a fatty acid ($C_{12-18}$) monoester of an adduct of 7 to 10 mol ethylene oxide with glycerol,

(E) 4 to 6% by weight of a water-insoluble perfume oil and

35 to 45% by weight water.


**Revendications**

1. Concentré de bain moussant en gel, caractérisé par la composition suivante:

(A) 35 à 45 % en poids de tensioactifs d'alkyléthersulfate de la formule $R^1$-$O(C_2H_4O)_n$-$SO_3Na$ , dans laquelle $R^1$ représente un groupement alkyle comportant 12 à 16 atomes de C et n est un nombre de 1 à 6.

(B) 5 à 15 % en poids d'un ou de plusieurs tensioactifs de polyglycoléther non ioniques de la formule $R^2$-A-$(C_2H_4O)_x$-H , dans laquelle $R^2$ représente un groupement alkyle, alcényle ou acyle comportant 12 à 18 atomes de C, A correspond à l'oxygène ou à un groupement -NH et x est un nombre de 3 à 9,

(C) 1 à 5 % en poids d'un ou de plusieurs tensioactifs amphotères ou zwitterioniques,

(D) 1 à 5 % en poids d'un monoester d'acide gras de glycérinepolyglycoléther ou d'un polylycoléther de mono- ou de diglycéride d'acide gras, à basé d'un acide gras comportant 12 à 18 atomes de C et 5 à 20 groupements glycoléther dans chaque cas.

(E) 2 à 10 % en poids d'une huile odoriférante insoluble dans l'eau et

30 à 50 % en poids d'eau.


2. Concentré de bain moussant en gel selon la revendication 1, caractérisé par la composition suivante:

(A) 35 à 40 % en poids de tensio-actif d'alkyléthersulfate de la formule $R^1$-O-$(C_2H_4O)_n$-$SO_3Na$, dans laquelle $R^1$ représente un groupement alkyle comportant 12 à 16 atomes de carbone et n est un nombre de 1 à 3,

(B1) 2 à 6 % en poids d'un tensioactif de polyglycoléther non ionique de la formule $R^2$-O-$(C_2H_4O)_x$-H dans laquelle $R^2$ représente, un groupement alkyle ou alcényle comportant 16 à 18 atomes de C et x est un nombre de 3 à 6,

(B2) 4 à 8 % en poids d'un tensioactif de polyglycoléther non ionique de la formule $R^3CONH$-$(C_2H_4O)_x$-H, dans laquelle $R^3CO$ représente un groupement acyle comportant 12 à 18 atomes de C x est un nombre de 3 à 6,

(C) 2 à 4 % on poids d'un tensioactif zwitterionique de la formule $R^4CONH$-$(CH_2)_3$-$N^{(+)}CH_3)_2CH_2COO^{(-)}$ ,dans laquelle $R^4CO$ représente un groupement acyle comportant 12 à 18 atomes de C,

(D) 2 à 4 % en poids d'un monoester (de $C_{12}$ à $C_{18}$) d'acide gras d'un produit d'addition de 7 à 10 moles d'oxyde d'éthylène à de la glycérine,

(E) 4 à 6 % en poids d'une huile odoriférante insoluble dans l'eau et

35 à 45 % en poids d'eau.